# FASCICULE DE BREVET EUROPEEN

(11) **EP 1 218 047 B1**
(45) Date de publication et mention de la délivrance du brevet: **16.03.2005**
(21) Numéro de dépôt: 00964301.6
(22) Date de dépôt: 15.09.2000
(51) Int. Cl.: A61M 5/32

(54) **DISPOSITIF D'INJECTION A USAGE UNIQUE**
INJEKTIONSVORRICHTUNG FÜR EINMALVERWENDUNG
DISPOSABLE INJECTION DEVICE

(30) Priorité: 07.10.1999 FR 9912501
(43) Date de publication de la demande: 03.07.2002
(73) Titulaire: SANOFI-SYNTHELABO, 75013 Paris (FR)
(72) Inventeur: BRUNEL, Marc, F-31000 Toulouse (FR)
(74) Mandataire: Sewell, Richard Charles
(86) Numéro de dépôt international: PCT/FR2000/002570
(87) Numéro de publication internationale: WO 2001/024856

(56) Documents cités:
- WO-A-93/02728
- FR-A- 2 770 405
- US-A- 5 137 516
- US-A- 5 147 325

## Description

L'invention concerne un dispositif d'injection à usage unique comportant une seringue pré-remplie dotée d'un nez antérieur portant une aiguille d'injection protégée par un capuchon de protection, et un corps de seringue logeant ladite seringue et doté de moyens de blocage en rotation et en translation de cette dernière.

A l'heure actuelle, à titre de sécurité, certains dispositifs d'injection du type ci-dessus décrit, tel le dispositif de US 5,137,516, comportent un embout de protection du capuchon ayant également pour fonction de faciliter le retrait du capuchon de protection.

Ces embouts de protection généralement adaptés pour venir s'emboîter sur l'extrémité antérieure du corps de seringue comportent, en outre, un manchon central adapté pour venir coiffer le capuchon de protection et doté de griffes de préhension qui viennent se clipser à l'arrière dudit capuchon.

Bien que dans la pratique de tels embouts de protection permettent de remplir les objectifs visés, ils présentent toutefois un inconvénient majeur du fait que le manchon central est emboîté en force sur le capuchon de protection, et tend, lors de cet emboîtement, à repousser ledit capuchon lors du passage du col d'étanchéité dont est doté classiquement le nez antérieur des seringues classiques.

Or, le fait de repousser ainsi le capuchon peut avoir deux conséquences fâcheuses. En effet, et en premier lieu, il peut conduire à détériorer l'extrémité de l'aiguille piquée classiquement dans le capuchon. De plus, il peut entraîner une rupture de l'étanchéité entre le capuchon de protection et le nez de seringue au niveau du col d'étanchéité de cette dernière.

La présente invention vise à pallier ces inconvénients et a pour principal objectif de fournir un dispositif d'injection doté d'un système de sécurité de protection du capuchon de protection permettant le retrait aisé de ce dernier, et non susceptible de détériorer l'aiguille et d'affecter l'étanchéité.

A cet effet, l'invention vise un dispositif d'injection tel que décrit dans le préambule ci-dessus, se caractérisant en ce que :
- il comprend un embout, dit embout à griffes, comportant une paroi frontale, et au moins deux pattes extensibles radialement s'étendant longitudinalement par rapport à la paroi frontale, aptes à coiffer le capuchon de protection et dotées d'au moins une griffe interne de préhension dudit capuchon,
- le corps de seringue présente une face frontale postérieure ouverte apte à permettre l'introduction de la seringue, et un tronçon antérieur opposé sécable délimité par une zone frangible ménagée de façon à être positionnée sensiblement au niveau du nez antérieur de la seringue logée dans ledit corps de seringue, ledit tronçon sécable comportant un col adapté pour resserrer les pattes de l'embout à griffes de façon que ces dernières assurent la préhension du capuchon de protection,
- lesdits tronçon sécable et embout à griffes étant dotés de moyens conjugués de blocage relatif en translation et en rotation de ces derniers.

Selon l'invention, et en premier lieu, l'embout à griffes est simplement positionné sur le capuchon de protection avant introduction de la seringue dans le corps de seringue, et vient réellement se resserrer sur ledit capuchon par déformation radiale des pattes au niveau du col dont est doté le tronçon sécable.

Cette solution qui consiste à emprisonner le capuchon de protection par déformation radiale des pattes de l'embout à griffes conduit à supprimer les risques de détérioration de l'aiguille et de rupture de l'étanchéité.

Toutefois, afin de garantir cette absence de risques, et de façon préférentielle, chaque griffe de préhension de l'embout à griffes est positionnée de façon à assurer la préhension du capuchon de protection sensiblement à l'avant du col d'étanchéité.

Selon l'invention, en outre, le tronçon sécable constitue un élément de protection présentant des avantages indéniables quant à la sécurité et à l'inviolabilité de la protection. De plus, du fait du blocage relatif en rotation et en translation de ce tronçon sécable et de l'embout à griffes, la rupture de la zone frangible permet de retirer simultanément lesdits tronçon sécable et embout à griffes et le capuchon de protection emprisonné dans ce dernier, conduisant ainsi à libérer directement l'aiguille d'injection.

Il est à noter, en outre, que la rupture de la zone frangible étant obtenue classiquement en imprimant un mouvement de rotation au tronçon sécable, le capuchon de protection est également amené à tourner lors de cette rupture et son décollage est ainsi grandement facilité.

Selon un mode de réalisation avantageux, les moyens de blocage relatif en rotation et en translation de l'embout à griffes et du tronçon sécable comprennent :
- une pluralité de crans longitudinaux ménagés sur au moins une des pattes de l'embout à griffes, de façon à être positionnés à l'avant du col du tronçon sécable du corps de seringue, lorsque la seringue est logée dans ce dernier,
- une pluralité de crans longitudinaux ménagés dans le tronçon sécable à l'avant du col de ce dernier,
- un épaulement de butée en translation de l'embout à griffes, formé par le col du tronçon sécable.

De plus, de façon avantageuse, les crans longitudinaux ménagés dans le tronçon sécable sont répartis sur la totalité de la périphérie de la face interne dudit tronçon sécable, de façon à s'affranchir de l'obligation d'indexer le positionnement angulaire relatif de l'embout à griffes et du tronçon sécable.

Par ailleurs, afin de faciliter le franchissement du col, et de façon avantageuse, les crans longitudinaux de chaque patte de l'embout à griffes sont ménagés sur un bossage doté d'une portion antérieure inclinée formant une rampe adaptée pour faciliter le passage du col.

Dans le même but, et de façon avantageuse, le col du tronçon sécable est constitué d'un bossage interne doté d'une portion postérieure inclinée formant rampe d'accès.

Par ailleurs, afin d'assurer un bon emprisonnement du capuchon de protection, le col est avantageusement ménagé de façon à être positionné au niveau des griffes de préhension de l'embout à griffes.

Selon un mode de réalisation avantageux, l'embout à griffes comporte quatre pattes longitudinales uniformément réparties par rapport à l'axe de la paroi frontale, deux desdites pattes diamétralement opposées comportant un bossage, les deux autres, dites de préhension, comportant au moins une griffe de préhension

De plus, de façon avantageuse, les pattes de préhension comprennent chacune deux griffes latérales de préhension.

D'autres caractéristiques, buts et avantages de l'invention ressortiront de la description détaillée qui suit en référence aux dessins annexés qui en représentent à titre d'exemple non limitatif un mode de réalisation préférentiel. Sur ces dessins :
- la figure 1 est une coupe longitudinale par un plan axial d'un dispositif d'injection conforme à l'invention, représenté avant utilisation,
- la figure 2 est une vue en perspective de l'étui protecteur de ce dispositif d'injection,
- la figure 3 est une coupe longitudinale par un plan axial A de cet étui protecteur,
- la figure 4 est une coupe longitudinale par un plan axial B de cet étui protecteur,
- la figure 5 est une coupe transversale par un plan C de cet étui protecteur,
- la figure 6 est une coupe transversale par un plan D de cet étui protecteur,
- la figure 7 est une coupe longitudinale par un plan axial E de la bague de verrouillage de ce dispositif d'injection,
- la figure 8 est une vue en perspective de cette bague de verrouillage,
- la figure 9 est une coupe longitudinale par un plan axial F de l'embout à griffes de ce dispositif d'injection,
- la figure 10 est une vue en perspective de cet embout à griffes,
- les figures 11a à 11f sont des vues schématiques représentant les étapes successives de fabrication de ce dispositif d'injection,
- la figure 12 est une coupe longitudinale par un plan axial de ce dispositif d'injection représentant la phase initiale de retrait du capuchon de protection, en vue de l'utilisation dudit dispositif d'injection,
- la figure 13 est une coupe longitudinale par un plan axial représentant le dispositif d'injection en fin d'injection,
- et la figure 14 est une coupe longitudinale par un plan axial représentant le dispositif d'injection dans sa position de protection après usage.

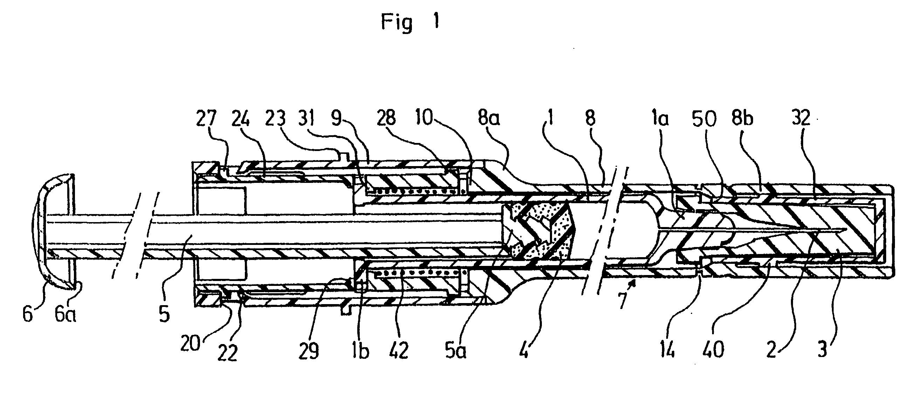

Le dispositif d'injection selon l'invention représenté à la figure 1 comprend, en premier lieu, une seringue pré-remplie 1 de type traditionnel, telle que par exemple réalisée en verre, comportant de façon classique un nez antérieur la sur lequel est montée une aiguille 2, et une collerette 1b au niveau de son extrémité postérieure.

Cette seringue 1 comporte également de façon classique un capuchon 3 de protection de l'aiguille 2 adapté pour être monté de façon étanche sur le nez antérieur 1a de ladite seringue.

Elle comprend également de façon classique un piston 4 délimitant la chambre remplie d'une dose de liquide, percé d'un alésage borgne taraudé dans lequel vient se visser l'extrémité filetée 5a d'une tige de piston 5 dotée au niveau de son extrémité opposée d'un poussoir 6.

Selon l'invention, ce poussoir 6 se présente sous la forme d'une coupelle présentant une tranche 6a à profil oblique à des fins explicitées plus loin.

Le dispositif d'injection selon l'invention comprend également un ensemble de protection de la seringue 1 adapté pour être entièrement pré-assemblé avant que ladite seringue initialement pré-remplie soit mise en place dans ledit ensemble de protection.

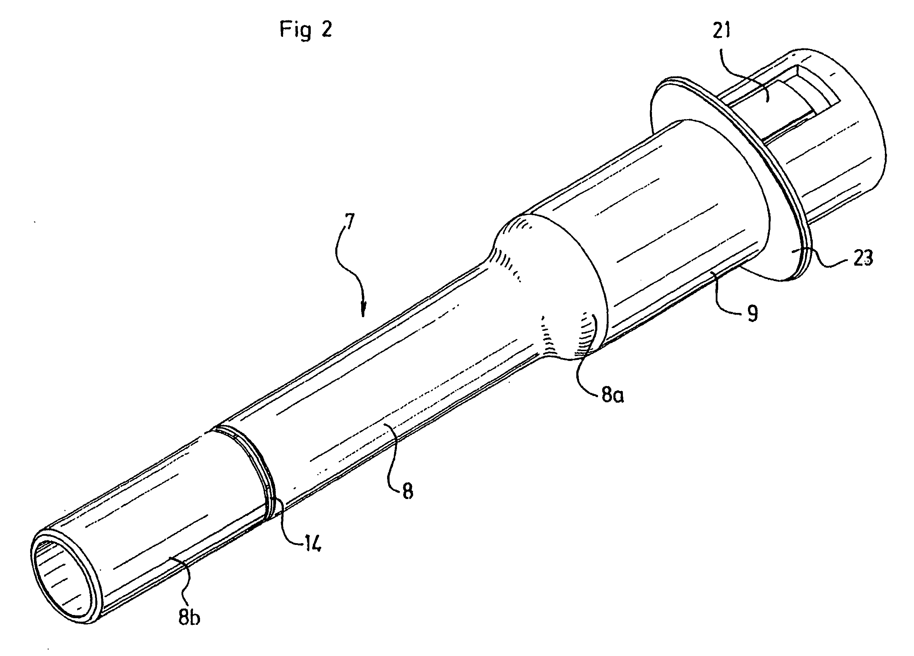

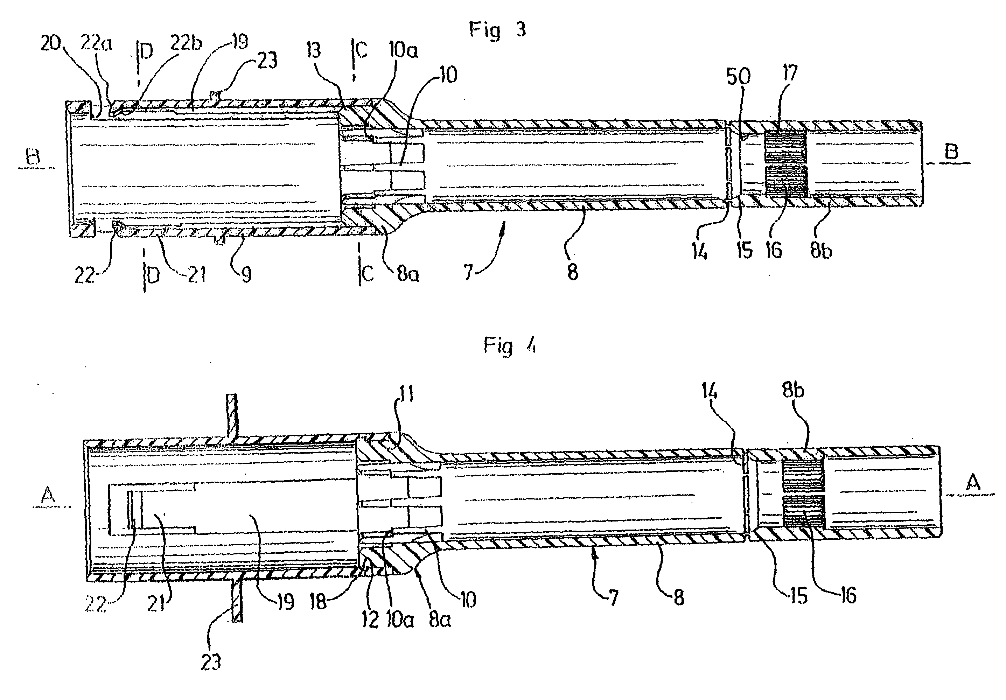

Cet ensemble de protection comprend en premier lieu un étui protecteur 7 représenté aux figures 2 à 6 composé de deux corps tubulaires antérieur 8 et postérieur 9 adaptés pour venir s'emmancher dans le prolongement l'un de l'autre.

Le corps antérieur 8 présente un diamètre interne conjugué du diamètre externe de la seringue 1 et une longueur adaptée pour loger l'aiguille 2 dotée de son capuchon de protection 3 et sensiblement 80% de la longueur de la seringue 1.

Au niveau de son extrémité postérieure, ce corps antérieur 8 comporte un tronçon postérieur 8a de forme externe sensiblement ovoïde de diamètres externes supérieurs au diamètre externe courant dudit corps antérieur, évidé intérieurement de façon à comporter des nervures longitudinales internes telles que 10 définissant un diamètre interne identique à celui du diamètre interne courant de ce corps antérieur 8.

Chacune de ces nervures 10 présente, en outre, un épaulement 10a délimitant une portion d'extrémité postérieure de diamètre interne sensiblement supérieur au diamètre de la seringue 1.

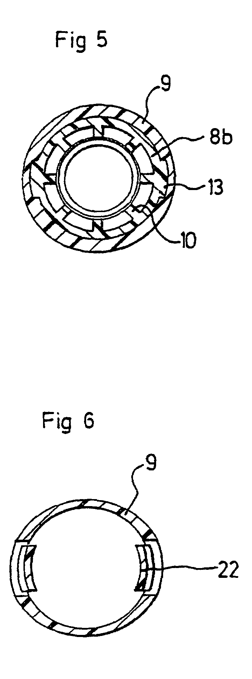

Côté externe, ce tronçon postérieur 8a comporte une gorge annulaire 11 délimitée par un jonc postérieur 12 de clipsage. De plus, tel que représenté à la figure 5, cette gorge 11 est interrompue par deux ergots tels que 13 diamétralement opposés faisant saillie radialement par rapport au jonc de clipsage 12.

Au niveau de son extrémité antérieure, le corps antérieur 8 comporte un tronçon antérieur 8b sécable délimité par une zone annulaire frangible 14 positionnée de façon à se trouver sensiblement au niveau du nez antérieur 1a de la seringue 1 une fois celle-ci mise en place.

Ce tronçon sécable 8b présente intérieurement au niveau de son extrémité postérieure un profil oblique 15 formant une rampe délimitant une portion postérieure constituant un col 50 de diamètre sensiblement inférieur à celui du diamètre interne courant du corps antérieur 8.

A l'avant de cette portion postérieure, le tronçon sécable 8b comporte, en outre, une pluralité de crans longitudinaux tels que 16 répartis sur la périphérie de la face interne dudit tronçon sécable et délimitant un diamètre interne identique à celui de ladite portion postérieure, de sorte que les fonds desdits crans définissent un épaulement 17 avec l'extrémité antérieure de cette portion postérieure.

En dernier lieu, concernant ce tronçon sécable 8b, la portion antérieure de ce dernier située à l'avant des crans 16 présente un diamètre interne identique au diamètre séparant le fond des crans.

Le corps postérieur 9 présente quant à lui une forme sensiblement ovoïde conjuguée de celle du tronçon postérieur 8a du corps antérieur 8, adaptée pour venir s'emmancher sur ledit tronçon postérieur 8a.

A cet effet, ce corps postérieur 9 comporte, en premier lieu, une gorge annulaire interne 18 agencée pour loger le jonc de clipsage 12. Il comporte, en outre, deux rainures longitudinales internes telle que 19 diamétralement opposées, aptes à loger chacune un ergot 13, de façon à assurer un blocage en rotation des deux corps 8, 9, lesdites rainures étant interrompues à faible distance de l'extrémité postérieure de ce corps postérieur 9, de façon que ce dernier présente un épaulement interne 20 au droit de l'extrémité de ces rainures 19.

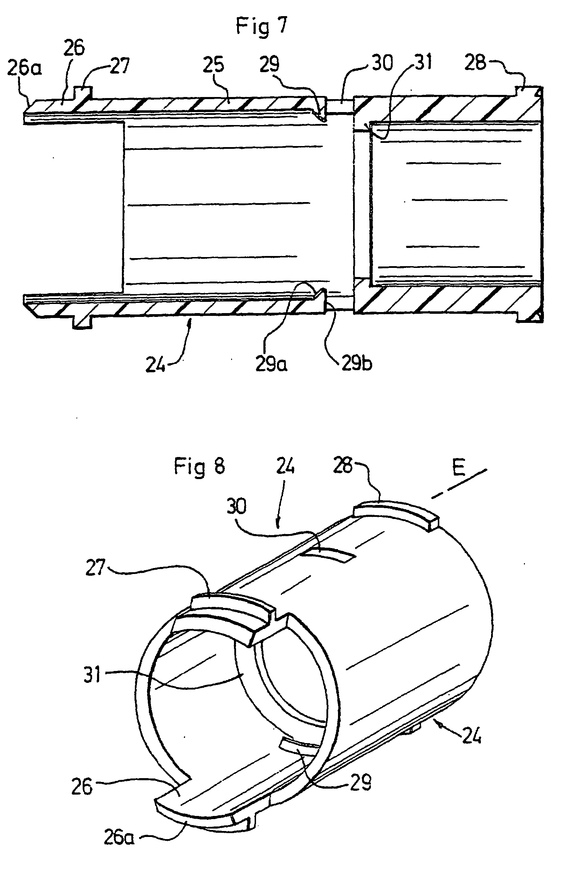

Tel que représenté à la figure 7, les rainures 19 sont ménagées selon le diamètre le plus grand du corps postérieur 9, de façon à minimiser l'épaisseur de la paroi dudit corps postérieur.

Le corps postérieur 9 présente, en outre, deux languettes déformables telles que 21 ménagées chacune dans une rainure 19 au niveau de l'extrémité postérieure de cette dernière et formées chacune par une découpe en forme de U ménagée dans la paroi dudit corps postérieur.

Chacune de ces languettes 21 comporte au niveau de son extrémité postérieure un crochet transversal 22 faisant saillie à l'intérieur du corps postérieur 9. Chacun de ces crochets comporte une face postérieure anti-retour 22a sensiblement radiale, et une face antérieure oblique 22b formant une rampe.

En dernier lieu, le corps postérieur 9 comporte une collerette externe classique 23 d'appui digital.

L'ensemble de protection comprend, en second lieu, une bague de verrouillage 24 de forme adaptée pour venir s'insérer dans le corps postérieur 9, en étant présentée en regard de la face antérieure de ce dernier.

Cette bague de verrouillage 24 de longueur adaptée pour s'insérer dans le corps postérieur 9 se présente sous la forme d'un manchon cylindrique 25 prolongé postérieurement par deux pattes telles que 26 diamétralement opposées en forme de secteur cylindrique.

Chacune de ces deux pattes 26 présente, en premier lieu, une face d'extrémité postérieure 26a à profil oblique complémentaire de celui de la tranche 6a du poussoir 6 de la tige de piston 5.

Chacune de ces pattes 26 comporte, en outre, sensiblement à mi-longueur, une nervure externe transversale 27, adaptée pour pouvoir coulisser entre deux des nervures 10 du corps postérieur 9.

Le manchon cylindrique 25 de cette bague de verrouillage 24 comporte, quant à lui, axées sur les mêmes génératrices que les nervures transversales 27, deux nervures transversales telles que 28 également adaptées pour coulisser entre les nervures 10 du corps postérieur 9, et ménagées au niveau de l'extrémité antérieure dudit manchon.

Ce manchon cylindrique 25 comprend également sensiblement à mi-longueur et dans l'alignement axial des nervures 27, 28 précitées, deux nervures internes telles que 29 diamétralement opposées à l'avant de chacune desquelles la paroi périphérique dudit manchon est percée d'une lumière telle que 30 permettant le démoulage de la contre-dépouille.

Chacune de ces nervures internes 29 présente une face postérieure 29a à profil oblique formant rampe et une face antérieure 29b radiale de blocage anti-retour.

Enfin, le manchon cylindrique 25 comprend à l'avant des lumières 30 et à une distance des nervures internes 29 conjuguée de l'épaisseur de la collerette 1b de la seringue 1, un épaulement interne annulaire 31.

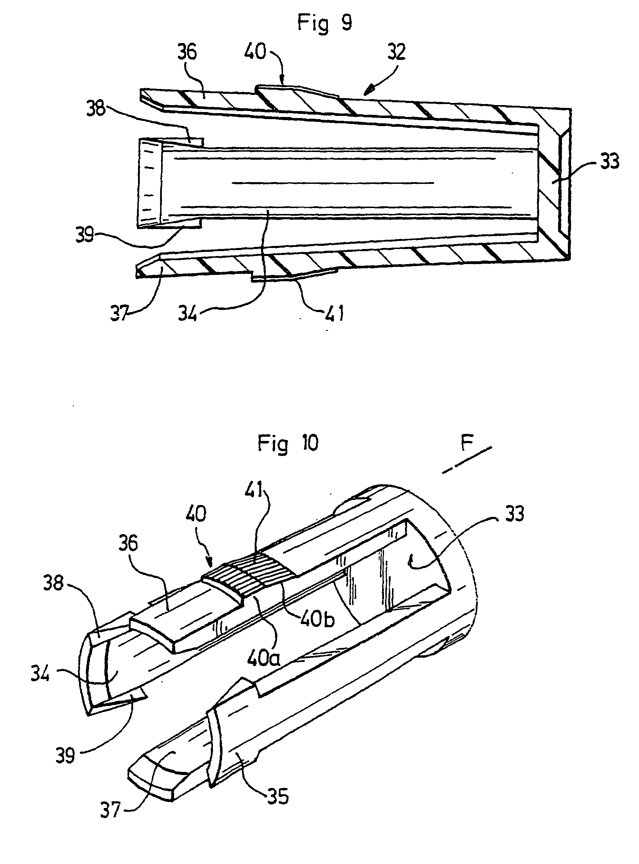

Le dispositif d'injection selon l'invention comprend, enfin, un embout à griffes 32 représenté aux figures 9 et 10 destiné à venir coiffer le capuchon de protection 3 et à entraîner le retrait de ce dernier après rupture de la zone frangible 14 du corps antérieur 8 de l'étui protecteur 7.

Cet embout à griffes 32 présente une paroi frontale cylindrique 33, de diamètre adapté pour pénétrer dans le tronçon sécable 8b, en périphérie de laquelle s'étendent sensiblement orthogonalement quatre pattes longitudinales distinctes 34, 35, 36, 37 uniformément réparties par rapport à l'axe de ladite paroi :
- deux pattes 34, 35 diamétralement opposées dotées au niveau de leur extrémité libre chacune de deux griffes latérales telles que 38, 39 adaptées pour pouvoir pénétrer dans le capuchon de protection 3,
- deux autres pattes 36, 37 diamétralement opposées présentant, en position intermédiaire de leur longueur, chacune un bossage externe tel que 40 présentant une face externe pourvue de crans longitudinaux tels que 41 conjugués des crans 16 du tronçon sécable 8b. De plus, chaque bossage 40 présente une portion postérieure cylindrique 40a précédée d'une portion antérieure inclinée 40b formant rampe.

Il est à noter, en outre, que tel que représenté à la figure 9, lors du moulage, les pattes 34-37 sont réalisées en position sensiblement "ouverte", c'est-à-dire inclinées vers l'extérieur par rapport à l'axe de la paroi frontale 33.

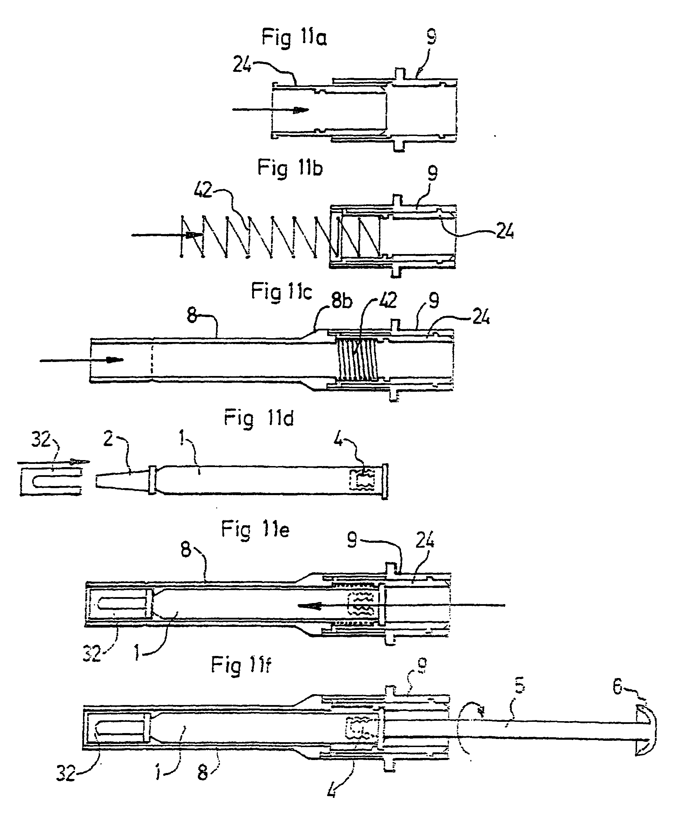

La fabrication du dispositif d'injection ci-dessus décrit ainsi que l'interconnexion des divers organes des éléments constitutifs est explicitée ci-dessous en référence aux figures 11a à 11f.

La première étape consiste à introduire la bague de verrouillage 24 à l'intérieur du corps postérieur 9 de l'étui protecteur 7, en présentant cette dernière en regard de la face antérieure dudit corps postérieur, jusqu'à amener les nervures 27 en butée contre l'épaulement 20 (figure 11a). Il est à noter que cette mise en place est autorisée par l'élasticité des languettes 21 et à la forme de la face antérieure 22b des nervures 22 qui forme une rampe autorisant le franchissement desdites nervures.

Il est également à noter qu'une fois la bague de verrouillage 24 mise en place, celle-ci est bloquée en rotation relativement au corps postérieur 9 du fait du positionnement des nervures 27 dans les rainures 19.

La deuxième étape consiste à introduire partiellement un ressort à spirale 42 à l'intérieur de la bague de verrouillage 24 en présentant ce dernier en regard de la face antérieure de ladite bague, jusqu'à amener une de ses extrémités en butée contre l'épaulement 31 (figure 11b).

La troisième étape consiste à venir emmancher le corps antérieur 8 sur le corps postérieur par coopération du jonc de clipsage 12 avec la gorge 18 (figure 11c). Au cours de cette opération, le ressort 42 se trouve automatiquement comprimé entre les épaulements 10a des nervures 10 et l'épaulement 31. De plus, les corps antérieur 8 et postérieur se trouvent bloqués en rotation relative du fait du positionnement des ergots 13 dans les rainures 19.

Au terme de ces trois opérations qui peuvent être facilement automatisées, on obtient un ensemble de protection entièrement pré-assemblé, à l'intérieur duquel peut ensuite être introduite la seringue pré-remplie 1 tel qu'explicité ci-dessous.

Préalablement à cette introduction, et tel que représenté à la figure 1 1d, l'embout à griffes 32 est positionné sur le capuchon de protection 3 de la seringue 1, à ce stade dépourvue de tige de piston 5. Lors de ce positionnement, les pattes 34-37 de l'embout à griffes 32 viennent uniquement se positionner autour du capuchon de protection 3, sans risque d'enfoncer ce dernier et d'endommager l'aiguille 2 et/ou de détruire l'étanchéité.

La seringue 1 munie de l'embout à griffes 32 est ensuite introduite dans le corps postérieur 9 de l'étui protecteur 7 jusqu'à amener la collerette 1b à se bloquer entre les nervures 29 et l'épaulement 31 (figure 11e). Il est à noter que cette introduction est autorisée par la faculté de se déformer des nervures 29 et à la forme de rampe de la face postérieure 29a de ces nervures 29 qui autorise le franchissement de ces dernières par la collerette 1b.

De plus, lors de cette introduction, l'embout à griffes 32 est amené à se resserrer sur le capuchon de protection 3 lors du passage des bossages 40 de ce dernier au niveau de la rampe 15 du tronçon sécable 8b et du col 50, passage en outre facilité par la forme de rampe de la portion antérieure 40b desdits bossages.

Il est à noter, en outre, que tel que représenté à la figure 1, lors de ce resserrement, les griffes 38, 39 viennent pénétrer dans le capuchon de protection 3 à l'avant du cône en verre dont sont munis classiquement les nez antérieurs des seringues classiques 1, destinées à assurer l'étanchéité. Ainsi, tout risque d'enfoncement du capuchon de protection 3 et donc d'endommagement de l'aiguille 2 et/ou de destruction de l'étanchéité se trouve écarté.

Une fois cette étape réalisée, il convient de noter que l'embout à griffes 32 et l'étui protecteur 7 sont bloqués en rotation relative du fait de la coopération des crans respectifs 16, 41 de ces derniers. De plus, d'une part, le capuchon de protection 3 est bloqué en rotation par rapport à l'embout à griffes 32 du fait de la pénétration dans ce dernier des griffes 38, 39, et d'autre part, cet embout à griffes 32, l'étui protecteur 7 et la bague de verrouillage 24 sont également bloqués en rotation relative tel qu'explicité ci-dessus.

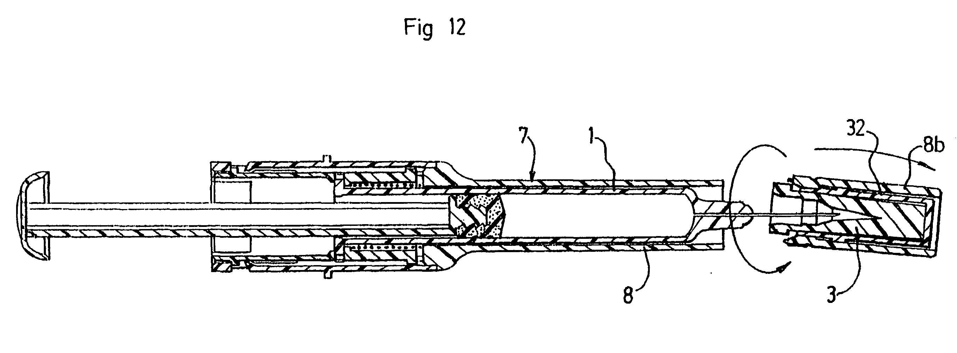

La dernière étape représentée à la figure 11f consiste enfin à solidariser de façon classique la tige de piston 5 au piston 4. On obtient alors un dispositif d'injection prêt à l'emploi dont l'utilisation est décrite ci-après en référence aux figures 12 à 14.

En premier lieu, tel que représenté à la figure 12, l'étape initiale consiste à rompre la zone frangible 14 en soumettant de façon classique le tronçon sécable 8 à un mouvement de rotation et en tirant celui-ci. Lors du mouvement, l'ensemble des éléments (capuchon de protection3, étui protecteur 7, bague de verrouillage 24 étant bloqués relativement en rotation, le capuchon de protection 3 est amené en premier lieu à tourner, facilitant le décollage de ce dernier, puis l'embout à griffes 32 est bloqué en translation par rapport au tronçon sécable 8b par butée des bossages 40 contre l'épaulement 17, de sorte que le capuchon de protection 3 est retiré simultanément avec l'embout à griffes 32 et ledit tronçon sécable.

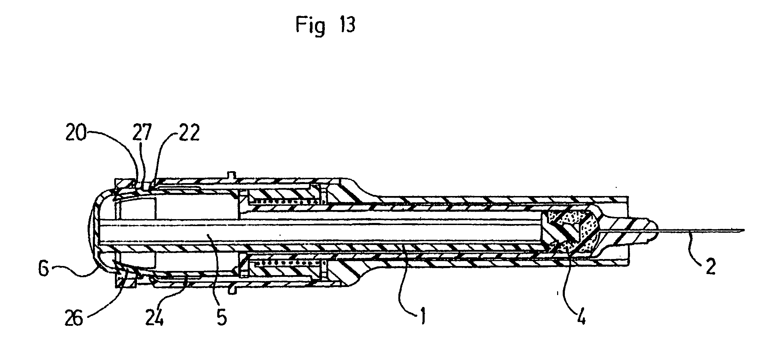

L'injection peut alors être réalisée de façon classique par action antagoniste sur le poussoir 6 et la collerette d'appui digital 23. En fin d'injection, tel que représenté à la figure 13, le bord profilé 6a du poussoir 6 vient coopérer avec le bord profilé 26a des pattes 26 de la bague de verrouillage 24, conduisant à déformer radialement vers l'intérieur lesdites pattes 26 jusqu'à entraîner la libération des nervures 27.

Il est à noter, en outre, que les nervures 27 étant disposées en position intermédiaire sur les pattes 26, ces dernières possèdent ainsi une rampe postérieure auxdites nervures qui permet d'absorber les tolérances de fabrication des seringues 1 et de garantir la délivrance de l'intégralité de la dose de liquide.

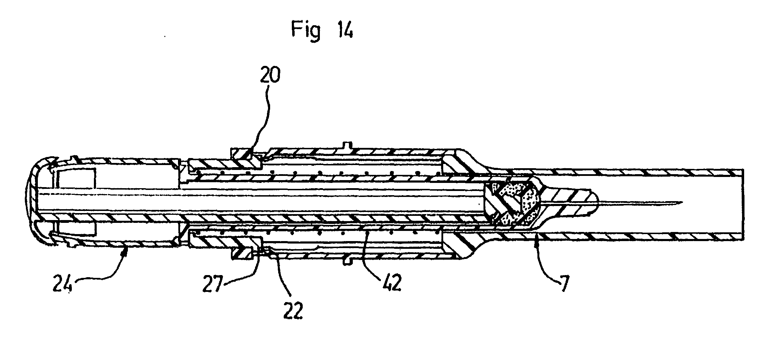

Tel que représenté à la figure 14, une fois l'injection terminée et l'effort sur le poussoir 6 supprimé, la bague de verrouillage 24 sollicitée par le ressort 42 est repoussée à l'intérieur de l'étui protecteur 7 entraînant la seringue 1, jusqu'à ce que les nervures 28 franchissent la rampe 22b des nervures 22 et viennent se bloquer entre lesdites nervures 22 et l'épaulement 20, interdisant un emploi ultérieur du dispositif d'injection.

En dernier lieu, et de façon avantageuse, le corps antérieur 8 de l'étui protecteur 7 est réalisé en un matériau translucide de façon à permettre de visualiser la seringue 1. Le corps postérieur 9 de cet étui protecteur est quant à lui réalisé en un matériau opaque de façon à masquer à la vue de l'utilisateur et du patient le mécanisme de déclenchement.

La bague de verrouillage 24 est quant à elle réalisée en un matériau de couleur différente de celui du corps postérieur 9 de façon à permettre d'identifier immédiatement les dispositifs d'injection déjà utilisés.

## Revendications

1. Dispositif d'injection à usage unique comportant une seringue pré-remplie (1) dotée d'un nez antérieur (1a) portant une aiguille (2) d'injection protégée par un capuchon de protection (3), et un corps de seringue (7) logeant ladite seringue et doté de moyens (24) de blocage en rotation et en translation de cette dernière,
ledit dispositif d'injection étant **caractérisé en ce que** :
- il comprend un embout (32), dit embout à griffes, comportant une paroi frontale (33), et au moins deux pattes (34-37) extensibles radialement s'étendant longitudinalement par rapport à la paroi frontale (33), aptes à coiffer le capuchon de protection (3) et dotées d'au moins une griffe interne (38, 39) de préhension dudit capuchon,
- le corps de seringue (7) présente une face frontale postérieure ouverte apte à permettre l'introduction de la seringue (1), et un tronçon antérieur opposé sécable (8b), délimité par une zone frangible (14) ménagée de façon à être positionnée sensiblement au niveau du nez antérieur (1a) de la seringue (1) logée dans ledit corps de seringue, ledit tronçon sécable comportant un col (50) adapté pour resserrer les pattes (34-37) de l'embout à griffes (32) de façon que ces dernières assurent la préhension du capuchon de protection (3),
- lesdits tronçon sécable (8b) et embout à griffes (32) étant dotés de moyens conjugués (16, 17, 40, 41) de blocage relatif en translation et en rotation de ces derniers.

2. Dispositif d'injection selon la revendication 1, **caractérisé en ce que** les moyens de blocage relatif en rotation et en translation de l'embout à griffes (32) et du tronçon sécable (8b) comprennent :
- une pluralité de crans longitudinaux (41) ménagés sur au moins une des pattes (36, 37) de l'embout. à griffes (32), de façon à être positionnés à l'avant du col (50) du tronçon sécable (8b) du corps de seringue (7), lorsque la seringue (1) est logée dans ce dernier,
- une pluralité de crans longitudinaux (16) ménagés dans le tronçon sécable (8b) à l'avant du col (50) de ce dernier,
- un épaulement (17) de butée en translation de l'embout à griffes (32) formé par ledit col (50).

3. Dispositif d'injection selon la revendication 2, **caractérisé en ce que** les crans longitudinaux (16) ménagés dans le tronçon sécable (8b) sont répartis sur la totalité de la périphérie de la face interne dudit tronçon sécable.

4. Dispositif d'injection selon l'une des revendications 2 ou 3, **caractérisé en ce que** les crans longitudinaux (41) de chaque patte (36, 37) de l'embout à griffes (32) sont ménagés sur un bossage (40) doté d'une portion antérieure (40b) formant une rampe adaptée pour faciliter le passage du col (50).

5. Dispositif d'injection selon l'une des revendications précédentes dans lequel le nez antérieur (1a) de la seringue (1) comporte un col externe d'étanchéité, **caractérisé en ce que** chaque griffe de préhension (38, 39) de l'embout à griffes (32) est positionnée de façon à assurer la préhension du capuchon de protection (3) sensiblement à l'avant aval dudit col d'étanchéité.

6. Dispositif d'injection selon l'une des revendications précédentes, **caractérisé en ce que** le col (50) du tronçon sécable (8b) est ménagé de façon à être positionné au niveau des griffes de préhension (38, 39) de l'embout à griffes (32).

7. Dispositif d'injection selon l'une des revendications précédentes, **caractérisé en ce que** le col (50) du tronçon sécable (8b) est constitué d'un bossage interne doté d'une portion postérieure inclinée (15) formant rampe d'accès.

8. Dispositif d'injection selon la revendication 4, **caractérisé en ce que** l'embout à griffes (32) comporte quatre pattes longitudinales (34-37) uniformément réparties par rapport à l'axe de la paroi frontale (33), deux desdites pattes (36, 37) diamétralement opposées comportant un bossage (40), les deux autres (34, 35), dites de préhension, comportant au moins une griffe de préhension (38, 39).

9. Dispositif d'injection selon la revendication 8, **caractérisé en ce que** les pattes de préhension (34, 35) comprennent chacune deux griffes latérales de préhension (38, 39).

## Patentansprüche

1. Injektionsvorrichtung für Einmalverwendung, die eine vorab gefüllte Spritze (1), die mit einer vorderen Nase (1a) versehen ist, die eine durch eine Schutzkappe (3) geschützte Injektionsnadel (2) trägt, und einen Spritzenkörper (7) aufweist, in welchem die Spritze untergebracht ist und welcher mit Einrichtungen (24) versehen ist, um eine Rotation und Translation dieser zu blockieren,
wobei die Injektionsvorrichtung **dadurch gekennzeichnet ist, daß**
- sie ein Stück (32), genannt Zangenstück, aufweist, das eine Stirnwand (33) und mindestens zwei sich in Längsrichtung bezüglich der Stirnwand (33) erstreckende, radial elastische Klauen (34-37) aufweist, die angepaßt sind, um die Schutzkappe (3) zu bedecken und mit mindestens einer inneren Kralle (38, 39) zum Greifen der Kappe versehen sind,
- der Spritzenkörper (7) eine offene hintere Stirnfläche, die angepaßt ist, um das Einführen der Spritze (1) zu erlauben, und einen gegenüberliegenden vorderen teilbaren Abschnitt (8b) aufweist, der durch eine zerbrechliche Zone (14) begrenzt ist, die so ausgebildet ist, dass sie im wesentlichen in Höhe der vorderen Nase (1a) der im Spritzenkörper untergebrachten Spritze (1) angeordnet ist, wobei der teilbare Abschnitt einen Hals (50) aufweist, der angepaßt ist, um die Klauen (34-37) des Zangenstücks (32) zu schließen, so daß letztere das Greifen der Schutzkappe (3) sicherstellen, und
- der teilbare Abschnitt (8b) und das Zangenstück (32) mit zusammenwirkenden Einrichtungen (16,17,40,41) zum Blockieren einer relativen Translation und Rotation dieser letzteren versehen sind.

2. Injektionsvorrichtung nach Anspruch 1, **dadurch gekennzeichnet, daß** die Einrichtungen zum Blockieren einer relativen Rotation und Translation des Zangenstücks (32) und des teilbaren Abschnitts (8b) aufweisen:
- mehrere Längskerben (41), die mindestens an einer der Klauen (36,37) des Zangenstücks (32) ausgespart sind, um vor dem Hals (50) des teilbaren Abschnitts (8b) des Spritzenkörpers (7) angeordnet zu sein, wenn die Spritze (1) darin untergebracht ist,
- mehrere Längskerben (16), die in dem teilbaren Abschnitt (8b) vor dessen Hals (50) ausgespart sind, und
- eine von dem Hals (50) gebildete Anschlagschulter (17) für die Translation des Zangenstücks (32).

3. Injektionsvorrichtung nach Anspruch 2, **dadurch gekennzeichnet, daß** die in dem teilbaren Abschnitt (8b) ausgesparten Längskerben (16) über den gesamten Umfang der Innenfläche des teilbaren Abschnitts verteilt sind.

4. Injektionsvorrichtung nach einem der Ansprüche 2 oder 3, **dadurch gekennzeichnet, daß** die Längskerben (41) jeder Klaue (36,37) des Zangenstücks (32) auf einer Erhebung (40) ausgespart sind, die mit einem vorderen Abschnitt (40b) versehen ist, der eine Rampe bildet, die angepaßt ist, um das Passieren des Halses (50) zu erleichtern.

5. Injektionsvorrichtung nach einem der vorstehenden Ansprüche, in welcher die vordere Nase (1a) der Spritze (1) einen äußeren Dichtungshals aufweist, **dadurch gekennzeichnet, daß** jede Greiferkralle (38,39) des Zangenstücks (32) so angeordnet ist, um das Greifen der Schutzkappe (3) im wesentlichen vor dem Dichtungshals sicherzustellen.

6. Injektionsvorrichtung nach einem der vorstehenden Ansprüche, **dadurch gekennzeichnet, daß** der Hals (50) des teilbaren Abschnitts (8b) so ausgespart ist, um in Höhe der Greiferkrallen (38,39) des Zangenstücks (32) angeordnet zu sein.

7. Injektionsvorrichtung nach einem der vorstehenden Ansprüche, **dadurch gekennzeichnet, daß** der Hals (50) des teilbaren Abschnitts (8b) aus einer inneren Erhebung besteht, die mit einem geneigten hinteren Abschnitt (15) versehen ist, der eine Zugangsrampe bildet.

8. Injektionsvorrichtung nach Anspruch 4, **dadurch gekennzeichnet, daß** das Zangenstück (32) vier längsverlaufende Klauen (34-37) aufweist, die gleichmäßig bezüglich der Achse der Stirnwand (33) verteilt sind, wobei zwei diametral gegenüberliegende dieser Klauen (36,37) eine Erhebung (40) und die zwei anderen Klauen (34,35), Greiferklauen genannt, mindestens eine Greiferkralle (38,39) aufweisen.

9. Injektionsvorrichtung nach Anspruch 8, **dadurch gekennzeichnet, daß** die Greiferklauen (34,35) jeweils zwei seitliche Greiferkrallen (38,39) aufweisen.

## Claims

1. A single-use injection device comprising a pre-filled syringe (1) provided with a front nose (1a) bearing an injection needle (2) which is protected by a protective cap (3), and a syringe body (7) which accommodates said syringe and is provided with means (24) for preventing the latter from moving in rotation and translation,
said injection device being **characterized in that**:
- it comprises an end piece (32), referred to as an end piece with claws, comprising a front wall (33), and at least two radially extendable tabs (34-37) extending longitudinally relative to the front wall (33), adapted to cap the protective cap (3) and which are provided with at least one inner claw (38, 39) for grasping said cap,
- the syringe body (7) has an open rear front face which adapted to allow the introduction of the syringe (1), and an opposite front section (8b), which can be separated and which is delimited by a frangible area (14) arranged so as to be positioned substantially at the front nose (1a) of the syringe (1) accommodated in said syringe body, said separable section comprising a neck (50) adapted to clamp the tabs (34-37) of the end piece (32) with claws such that these latter ensure that the protective cap (3) is grasped,
- said separable section (8b) and end piece (32) with claws being provided with means in correspondence (16, 17, 40, 41) to prevent relative translation and rotation of these latter.

2. An injection device according to claim 1, **characterized in that** the means for preventing relative rotation and translation of the end piece (32) with claws and of the separable section (8b) comprise:
- a plurality of longitudinal notches (41) provided on at least one of the tabs (36, 37) of the end piece with claws (32), so as to be positioned ahead the neck (50) of the separable section (8b) of the syringe body (7), when the syringe (1) is accommodated in the latter,
- a plurality of longitudinal notches (16) provided on the separable section (8b) ahead of the neck (50) of the latter,
- a shoulder (17) for translational abutment of the end piece with claws (32) formed by said neck (50).

3. An injection device according to claim 2, **characterized in that** the longitudinal notches (16) which are provided on the separable section (8b) are distributed around the entire periphery of the inner surface of said separable section.

4. An injection device according to one of claims 2 or 3, **characterized in that** the longitudinal notches (41) of each tab (36, 37) of the end piece with claws (32) are advantageously disposed on a boss (40) provided with a front portion (40b) forming a ramp which is adapted to facilitate passage of the neck (50).

5. An injection device according to one of the preceding claims in which the front nose (1a) of the syringe (1) comprises an outer sealing neck, **characterized in that** each grasping claw (38, 39) of the end piece (32) with claws is positioned so as to ensure that the protective cap (3) is grasped substantially downstream ahead of the sealing neck.

6. An injection device according to one of the preceding claims, **characterized in that** the neck (50) of the separable section (8b) is provided so as to be positioned adjacent the grasping claws (38, 39) of the end piece (32) with claws.

7. An injection device according to one of the preceding claims, **characterized in that** the neck (50) of the separable section (8b) is constituted by an inner boss provided with an inclined rear portion (15) forming an access ramp.

8. An injection device according to claim 4, **characterized in that** the end piece (32) with claws comprises four longitudinal tabs (34-37) which are distributed uniformly relative to the axis of the front wall (33), two of said tabs (36, 37) which are diametrically opposite comprising a boss (40), and the two others (34, 35), referred to as grasping tabs, comprising at least one grasping claw (38, 39).

9. An injection device according to claim 8, **characterized in that** the grasping tabs (34, 35) each comprise two lateral grasping claws (38, 39).
